(19) Europäisches Patentamt / European Patent Office / Office européen des brevets

(11) **EP 2 791 493 B1**

(12) **EUROPÄISCHE PATENTSCHRIFT**

(45) Veröffentlichungstag und Bekanntmachung des Hinweises auf die Patenterteilung:
**17.01.2024   Patentblatt 2024/03**

(21) Anmeldenummer: **12779013.7**

(22) Anmeldetag: **17.10.2012**

(51) Internationale Patentklassifikation (IPC):
**F02D 41/14** *(2006.01)*      **F02D 41/22** *(2006.01)*
**G01N 33/00** *(2006.01)*

(52) Gemeinsame Patentklassifikation (CPC):
**G01N 33/0006; F02D 41/1454; F02D 41/1458; F02D 41/1495;** F02D 41/222; F02D 2041/1432; F02D 2041/1433

(86) Internationale Anmeldenummer:
**PCT/EP2012/070531**

(87) Internationale Veröffentlichungsnummer:
**WO 2013/087262 (20.06.2013 Gazette 2013/25)**

(54) **VERFAHREN UND VORRICHTUNG ZUR DYNAMIKÜBERWACHUNG VON GAS-SENSOREN**

METHOD AND APPARATUS FOR MONITORING EXHAUST GAS SENSOR DYNAMICS

PROCÉDÉ ET DISPOSITIF PERMETTANT DE SURVEILLER LA DYNAMIQUE DES CAPTEURS DE GAZ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **12.12.2011   DE 102011088296**

(43) Veröffentlichungstag der Anmeldung:
**22.10.2014   Patentblatt 2014/43**

(73) Patentinhaber: **Robert Bosch GmbH 70442 Stuttgart (DE)**

(72) Erfinder: **MICHALSKE, Andreas 70806 Kornwestheim (DE)**

(56) Entgegenhaltungen:
**EP-A2- 1 074 718        EP-A2- 2 336 532
DE-A1-102008 040 737    DE-A1-102008 042 549
DE-A1-102009 028 367    US-B1- 6 382 198**

EP 2 791 493 B1

**Beschreibung**

Stand der Technik

Die Erfindung betrifft ein Verfahren zur Dynamiküberwachung von Gas-Sensoren einer Brennkraftmaschine, welche beispielsweise als Abgassonden im Abgaskanal einer Brennkraftmaschine als Teil eines Abgasüberwachungs- und -minderungssystems oder als Gaskonzentrationssensoren in einer Zuluftführung der Brennkraftmaschine angeordnet sind, wobei die Gas-Sensoren abhängig von Geometrie, Messprinzip, Alterung oder Verschmutzung ein Tiefpassverhalten aufweisen, wobei bei einer Änderung der zu erfassenden Gaszustandsgröße auf Grund eines Vergleiches eines modellierten und eines gemessenen Signals eine Dynamikdiagnose durchgeführt wird und wobei das gemessene Signal ein Istwert eines Ausgangssignals des Gas-Sensors und das modellierte Signal ein Modellwert ist.

Zur Reduktion der Emissionen in PKW mit Ottomotoren werden üblicherweise 3-Wege-Katalysatoren als Abgasreinigungsanlagen verwendet, die nur dann ausreichend Abgase konvertieren, wenn das Luft-Kraftstoffverhältnis λ mit hoher Präzision eingeregelt wird. Zu diesem Zweck wird das Luft-Kraftstoffverhältnis λ mittels einer der Abgasreinigungsanlage vorgelagerten Abgassonde gemessen. Das Speichervermögen einer derartigen Abgasreinigungsanlage für Sauerstoff wird dazu ausgenutzt, in Magerphasen Sauerstoff aufzunehmen und in Fettphasen wieder abzugeben. Hierdurch wird erreicht, dass oxidierbare Schadgaskomponenten des Abgases konvertiert werden können. Eine der Abgasreinigungsanlage nachgeschaltete Abgassonde dient dabei der Überwachung der Sauerstoff-Speicherfähigkeit der Abgasreinigungsanlage. Die Sauerstoff-Speicherfähigkeit muss im Rahmen der On-Board-Diagnose (OBD) überwacht werden, da sie ein Maß für die Konvertierungsfähigkeit der Abgasreinigungsanlage darstellt. Zur Bestimmung der Sauerstoff-Speicherfähigkeit wird entweder die Abgasreinigungsanlage zunächst in einer Magerphase mit Sauerstoff belegt und anschließend in einer Fettphase mit einem im Abgas bekannten Lambdawert unter Berücksichtigung der durchtretenden Abgasmenge entleert oder die Abgasreinigungsanlage zunächst in einer Fettphase von Sauerstoff entleert und anschließend in einer Magerphase mit einem im Abgas bekannten Lambdawert unter Berücksichtigung der durchtretenden Abgasmenge aufgefüllt. Die Magerphase wird beendet, wenn die der Abgasreinigungsanlage nachgeschaltete Abgassonde den Sauerstoff detektiert, der nicht mehr von der Abgasreinigungsanlage gespeichert werden kann. Ebenso wird eine Fettphase beendet, wenn die Abgassonde den Durchtritt von fettem Abgas detektiert. Die Sauerstoff-Speicherfähigkeit der Abgasreinigungsanlage entspricht der während der Fettphase zur Entleerung zugeführten Menge an Reduktionsmittel bzw. während der Magerphase zur Auffüllung zugeführten Menge an Sauerstoff. Die genauen Mengen werden aus dem Signal der vorgelagerten Abgassonde und dem aus anderen Sensorsignalen ermittelten Abgasmassenstrom berechnet.

Nimmt die Dynamik der vorgelagerten Abgassonde ab, z.B. auf Grund von Verschmutzungen oder Alterung, so kann das Luft-Kraftstoff-Verhältnis nicht mehr mit der erforderlichen Präzision eingeregelt werden, so dass die Konvertierungsleistung der Abgasreinigungsanlage nachlässt. Weiterhin können sich Abweichungen in der Diagnose der Abgasreinigungsanlage ergeben, die dazu führen können, dass eine an sich korrekt arbeitende Abgasreinigungsanlage fälschlich als nicht funktionsfähig bewertet wird. Der Gesetzgeber verlangt eine Diagnose der Sondeneigenschaften während des Fahrbetriebs, um sicherzustellen, dass das geforderte Luft-Kraftstoffverhältnis weiterhin ausreichend genau eingestellt werden kann, die Emissionen die zulässigen Grenzwerte nicht überschreiten und die Abgasreinigungsanlage korrekt überwacht wird. Die OBDII-Bestimmungen verlangen, dass Lambdasonden und andere Abgassonden nicht nur bezüglich ihrer elektrischen Funktionstüchtigkeit überwacht werden, sondern auch hinsichtlich ihres Ansprechverhaltens, d.h. es muss eine Verschlechterung der Sonden-Dynamik erkannt werden, die sich durch eine vergrößerte Zeitkonstante und/ oder Totzeit bemerkbar machen kann. Tot- und Verzögerungszeiten zwischen einer Veränderung der Abgaszusammensetzung und deren Erkennung müssen on-board darauf geprüft werden, ob sie für die Anwenderfunktionen, d.h. für Steuer-, Regel- und Überwachungsfunktionen, die das Sondensignal verwenden, noch zulässig sind. Als Kenngröβen für die Dynamikeigenschaften von Abgassensoren werden typischerweise die Totzeit von einer Gemischänderung bis zur Signalflanke und eine bestimmte Anstiegszeit, z.B. von 0 % auf 63 % oder von 30 % auf 60 % eines Signalhubs, verwendet. Die Totzeit enthält auch die Gaslaufzeit vom Motorauslass bis zur Sonde und verändert sich demnach insbesondere bei einer Manipulation des Sensoreinbauortes.

Bei Dieselmotoren werden als Gas-Sensoren oder Gaskonzentrationssensoren Breitband-Lambdasonden und, in Verbindung mit SCR-Katalysatoren, auch $NO_x$-Sensoren eingesetzt. Letztere liefern zusätzlich ebenfalls ein $O_2$-Signal. Das $O_2$-Signal von Breitband-Lambdasonde oder $NO_x$-Sensor wird beim Dieselmotor nicht nur für den Betrieb von Abgasnachbehandlungseinrichtungen verwendet, sondern auch für die innermotorische Emissionsreduktion. Die gemessene $O_2$-Konzentration im Abgas bzw. das gemessene Lambdasignal wird genutzt, um das Luft-Kraftstoffgemisch dynamisch genau einzustellen und so die Streuungen der Rohemissionen zu minimieren. Bei Dieselmotoren mit $NO_x$-Speicherkatalysator (NSC) wird je eine Breitband-Lambdasonde vor und nach Katalysator für eine zuverlässige Darstellung des Fettbetriebs zur Regeneration benötigt. Innermotorische Emissionsreduktion und NSC-Betrieb stellen ebenfalls bestimmte Mindestanforderungen an die Dynamikeigenschaften der $O_2$-Sonde. Die Anstiegszeit des $O_2$-Signals wird heutzutage beim Übergang von Last nach Schub überwacht, d.h. beim Anstieg von einem bestimmten Prozentsatz unter dem normalen O₂-Gehalt von Luft auf 21 %. Erreicht das Sensorsignal nach einer Maximalzeit nicht einmal einen bestimmten Zwischenwert, wird dies als Totzeitfehler interpretiert. Bei Dieselmotoren mit NO$_x$-Speicher-katalysator (NSC) wird daneben üblicherweise das Ansprechverhalten der Lambdasonden vor und nach Katalysator verglichen.

Für kommende Fahrzeuggenerationen bzw. Modelljahre ist zu erwarten, dass auch eine Überwachung der Sensordynamik bei fallender O₂-Konzentration gefordert wird. Außerdem wird es bei Hybridfahrzeugen künftig keine Schubphasen mehr geben und somit keine Phasen mit einer konstanten O₂-Konzentration von 21 %. Erste Lösungsansätze für diese Zusatzanforderungen sind die aktive Überwachung in der DE 10 2008 001 121 A1 sowie die beobachterbasierte Methode in der DE 10 2008 040 737 A1.

Aus der DE 10 2008 040 737 A1 ist ein Verfahren zur Überwachung von dynamischen Eigenschaften einer Breitband-Lambdasonde bekannt, wobei mittels der Breitband-Lambdasonde ein gemessenes Lambdasignal bestimmt wird, das einer SauerstoffKonzentration im Abgas einer Brennkraftmaschine entspricht, wobei der Brennkraftmaschine ein Beobachter zugeordnet ist, der aus Eingangsgrößen ein modelliertes Lambdasignal erzeugt und wobei aus der Differenz des modellierten Lambdasignals und des gemessenen Lambdasignals oder aus der Differenz aus einem aus dem modellierten Lambdasignal abgeleiteten Signal und einem aus dem gemessenen Lambdasignal abgeleiteten Signal ein Schätzfehler-Signal als Eingangsgröße eines in dem Beobachter einem Modell vorgeschalteten Reglers gebildet wird. Dabei ist vorgesehen, dass ein Maß für die durch eine Totzeit und eine Reaktionszeit charakterisierten dynamischen Eigenschaften der Breitband-Lambdasonde aus einer Bewertung des Schätzfehler-Signals oder einer daraus abgeleiteten Größe bestimmt wird und dass das Maß für die dynamischen Eigenschaften mit vorgegebenen Grenzwerten verglichen wird um zu bewerten, inwiefern die dynamischen Eigenschaften der Breitband-Lambdasonde für einen vorgesehenen Betrieb der Brennkraftmaschine ausreichen.

In der DE 10 2008 001 569 A1 wird zudem ein Verfahren und eine Vorrichtung zur On-line-Adaption eines LSU-Dynamikmodells beschrieben. Konkret betrifft die Schrift ein Verfahren und eine Vorrichtung zur Adaption eines Dynamikmodells einer Abgassonde, die Bestandteil eines Abgaskanals einer Brennkraftmaschine ist und mit der ein Lambdawert zur Regelung einer Luft-Kraftstoff-Zusammensetzung bestimmt wird, wobei in einer Steuereinrichtung bzw. in einer Diagnoseeinrichtung der Brennkraftmaschine parallel dazu ein simulierter Lambdawert berechnet wird und von einer Anwenderfunktion sowohl der simulierte als auch der gemessene Lambdawert verwendet wird. Dabei ist vorgesehen, dass im laufenden Fahrzeugbetrieb durch Auswerten einer Signaländerung bei Anregung des Systems ein Sprungverhalten der Abgassonde bestimmt und anhand dieser Ergebnisse das Dynamikmodell der Abgassonde adaptiert wird.

Für die Identifikation der Sensoreigenschaften wird dabei auf bekannte Funktionen zur Dynamiküberwachung von Breitband-Lambdasonden zurückgegriffen. Für andere Gaskonzentrationssignale von Abgassensoren, z.B. für ein NO$_x$-Signal, gelten vergleichbare Anforderungen wie für Os-Signale bzw. -Sensoren. Ähnlichkeiten zwischen den Überwachungsfunktionen sind daher anzunehmen.

Das Verfahren nach der DE 10 2008 001 121 A1 ist eine aktive Überwachung. Sie beinhaltet eine Anregung durch eine Testeinspritzung, die sowohl den Kraftstoffverbrauch als auch die Emissionen erhöht. Das Verfahren nach der DE 10 2008 040 737 A1 arbeitet zwar passiv, setzt aber einen sogenannten Beobachter voraus, dessen Applikation aufwendig ist. Außerdem zielen beide Verfahren primär auf die Erkennung von größeren Totzeitänderungen ab.

Ferner ist aus der DE 10 2008 042 549 A1 ein Verfahren zur Dynamiküberwachung bekannt.

Es besteht daher die Aufgabe, eine Dynamiküberwachung für Gas-Sensoren bereitzustellen, die zumindest einen Teil folgender Anforderungen erfüllt:

> Besondere Eignung für die Diagnose bzw. Identifikation von Anstiegszeiten,
> Einheitliches Überwachungsprinzip für Zu- und Abnahme einer Gaskonzentration,
> Passives Verfahren, d.h. ohne Eingriff in Luft- oder Kraftstoffsystem des Verbrennungsmotors,
> Anwendbar auch bei Fahrzeugen ohne Schubphasen und ohne Leerlauf (z.B. bei Hybridfahrzeugen),
> Hohe Verfügbarkeit in den relevanten Zertifizierungszyklen,
> Hohe Robustheit gegenüber Störungen und
> Geringe Komplexität sowie kleiner Applikationsaufwand.

Offenbarung der Erfindung

Die das Verfahren betreffende Aufgabe wird dadurch gelöst, dass das Ausgangssignal des Gas-Sensors mit einem Hochpassfilter gefiltert und bei einer Änderung der zu messenden Gaszustandsgröße, wie auch einer Gaskonzentration, höherfrequente Signalanteile ausgewertet werden. Eine Änderung kann dabei durch eine Anregung der Brennkraftmaschine erfolgen. Mit diesem Verfahren lassen sich Änderungen hinsichtlich der Dynamik bei Gas-Sensoren nachweisen und quantifizieren. Gas-Sensoren im Sinne der Erfindung sind Sensoren, die Zustände eines Gases messen bzw. Änderungen detektieren können. Der Zustand des Gases kann dabei durch eine Temperatur des Gases, einen Gasdruck, einen Gas-Massenstrom und/ oder eine Konzentration eines bestimmten Gasanteils, z.B. einen Sauerstoff-gehalt oder $NO_x$-Gehalt, beschrieben sein. Gas-Sensoren weisen ein typisches Tiefpassverhalten auf, das u.a. von der Geometrie ihres Aufbaus abhängt. Zudem können derartige Sensoren aufgrund von Alterung oder äußeren Einflüssen (z.B. infolge Verrußung bei Dieselmotoren) ihr Ansprechverhalten ändern. Im Zeitbereich äußert sich die abnehmende Grenzfrequenz in einer größeren Anstiegszeit, d.h. bei unveränderter Anregung werden die Signalflanken flacher. Schaltet man daher mit der Sonde einen geeigneten Hochpassfilter, z.B. einen Hochpassfilter 1. Ordnung, in Reihe, dann kann man bei steilen Änderungen der zu messenden Gaszustandsgröße, wie etwa der Gaskonzentration, am Ausgangssignal des Hochpasses erkennen, ob die Grenzfrequenz des Tiefpasses größer oder kleiner als die Grenzfrequenz das Hochpasses ist. Reagiert der Sensor infolge Alterung oder äußerer Einflüsse träge, werden bei Änderungen der Gaszustandsgrößen nur noch geringe oder keine höherfrequenten Signalanteile bestimmt. Weist der Sensor ein hohes Maß an Dynamik auf, wirkt sich dies auf einen relativ großen höherfrequenten Signalanteil aus, so dass mit diesem Merkmal eine Dynamikdiagnose realisiert werden kann. Mit dem vorgestellten Verfahren kann ein einheitliches Überwachungsprinzip für Zu- und Abnahme einer Gaszustandsgröße, z.B. einer Gaskonzentration, bereitgestellt werden, welches passiv ausgelegt ist, d.h. ohne einen Eingriff in das Luft- oder Kraftstoffsystem der Brennkraftmaschine auskommt, wie dies bei bisherigen Dynamik-Diagnoseverfahren der Fall ist. Es weist einerseits eine hohe Robustheit gegenüber Störungen auf. Andererseits zeichnet sich das Verfahren durch seine geringe Komplexität sowie durch einen kleinen Applikationsaufwand aus. Die eingangs erwähnten Anforderungen in der Aufgabenstellung können daher gleichzeitig erfüllt werden.

Erfindungsgemäß wird bei einer Änderung eines Luft-Kraftstoff-Verhältnisses eines der Brennkraftmaschine zugeführten Luft-Kraftstoffgemischs die Dynamikdiagnose des Gas-Sensors durchgeführt. Dabei werden die höherfrequenten Signalanteile ausgewertet.

Damit zwischen einem langsamen Sensor und einer unzureichenden Anregung unterschieden werden kann, muss die Änderungsgeschwindigkeit der zu messenden Gaszustandsgröße beurteilt werden, ohne dabei das Signal des zu überwachenden Sensors selbst zu verwenden. Erfindungsgemäß ist daher vorgesehen, dass die höherfrequenten Signalanteile des Gas-Sensors mit entsprechend hochpassgefilterten Ausgangssignalen aus einem Modell des Gas-Sensors verglichen werden und anhand des Vergleichs auf die Dynamik des Gas-Sensors geschlossen wird. Im Falle des $O_2$-Signals einer Breitband-Lambdasonde oder eines $NO_x$-Sensors werden dazu die Luft- und die Kraftstoffmasse als Eingangsgrößen dem Modell zugeführt, das die Restsauerstoffkonzentration im Abgas vorhersagt. Das Modell bildet dabei den Gas-Sensor mit seinem typischen Tiefpassverhalten, üblicherweise kombiniert mit einer Totzeit, ab. Durch einen Vergleich der beiden Hochpass-Ausgänge kann dann auf die Funktionstüchtigkeit des realen Sensors geschlossen werden. Im Falle einer anderen Gaskonzentration, wie beispielsweise eines $NO_x$-Signals, kann es erforderlich werden, ein zusätzliches Rohemissionsmodell zu verwenden.

Erfindungsgemäß ist vorgesehen, dass sowohl die höherfrequenten Signalanteile des Gas-Sensors als auch die des Modells quadriert sowie integriert und damit höherfrequente Energieanteile berechnet werden, und anschließend diese Energieanteile ins Verhältnis gesetzt werden und anhand des derart berechneten Energieverhältnisses auf das Dynamikverhalten des Gas-Sensors geschlossen wird. Je kleiner die Fläche unter dem quadrierten Ausgangssignal des Hochpasses ist, desto langsamer ist die Sonde bzw. die Anregung.

Damit multiplikative Fehler des Gas-Sensors und/ oder des Modells bzw. seiner Eingangssignale den Signalvergleich nicht verfälschen, wird bevorzugt eine Normierung der jeweiligen Energieanteile durchgeführt. Additive Fehler des Gas-Sensors oder des Modells bzw. seiner Eingangssignale wirken sich nicht aus, weil ein Hochpass den Gleichanteil eines Signals unterdrückt.

Bei der Ermittlung der Signalenergien kann vorgesehen sein, dass die Integration der höherfrequenten Signalanteile mittels einer für beide Signale individuellen Integrationsdauer $\Delta t$ durchgeführt wird, wobei der Zeitpunkt für den Start der Integration der beiden Signale sowohl bei einer ansteigenden Signalflanke des Ausgangssignals als auch bei einer fallenden Signalflanke getriggert wird. Falls steigende und fallende Flanken des Sensorsignals nicht getrennt überwacht werden müssen, kann das erfindungsgemäße Verfahren vereinfacht werden. Es ist dann alternativ möglich, die Integration in beiden Pfaden zu einem beliebigen Zeitpunkt zu starten und für die Dauer $\Delta t$ auszuführen. Vorausset-zung ist lediglich eine ausreichende Anregung durch Änderungen der zu messenden Gaszustandsgröße, wie etwa der Gaskonzentration, welche beispielsweise von wechselnden Motorbetriebspunkten herrühren fallende und steigende Flanken umfassen. Für die Normierung ist dann das jeweilige Maximum und Minimum während $\Delta t$ an beiden Hochpas-seingängen zu erfassen.

Eine bevorzugte Verfahrensvariante sieht vor, dass, ausgehend von einem bestimmten Stationärpunkt, bei Einsetzen der Signalflanken ein applizierbarer Signalhub abgewartet wird, bevor die Integration gestartet wird. Ein kurzzeitiger Stationärbetrieb vor der Überwachung stellt sicher, dass die Signalflanken von Sonde und Modell auch bei Totzeitunterschieden von derselben Anregung herrühren. Dies ist insbesondere dann wichtig, wenn der Einbauort der Abgassonde manipuliert wurde.

Wird das Energieverhältnis mit einem applizierbaren Schwellwert verglichen, der die Dynamik eines grenzwertigen Sensors repräsentiert, wobei der Schwellwert und eine im Modell für den Sensor gewählte Modell-Zeitkonstante

$T_M$ voneinander abhängig sind, kann sehr einfach eine Dynamik-Diagnose durchgeführt werden. Entspricht $T_M$ bereits einer grenzwertigen Sensordynamik, dann liegt der passende Schwellwert bei 1, so dass seine Applikation entfällt. Entspricht die Modell-Zeitkonstante $T_M$ dagegen einem ausreichend schnellen Gas-Sensor, z.B. einem Nominal-Sensor, dann liegt die Überwachungsgrenze für das Energieverhältnis unter 1. Für den Fall, dass das Energieverhältnis $\geq$ dem Schwellwert ist, ist der Sensor als i.O. zu betrachten, im Fall, dass das Energieverhältnis $\leq$ dem Schwellwert ist, ist der Sensor als fehlerhaft zu betrachten.

Die Kenngrößen für die Dynamik des Sensors hängen in der Regel vom Gasmassenstrom, dem Gasvolumenstrom bzw. von der Gasgeschwindigkeit ab. Soll die Diagnose in einem großen Motorbetriebsbereich eine hohe Trennschärfe aufweisen, dann ist es zweckmäßig, dass auch die verschiedenen Filterzeitkonstanten und/ oder der Schwellwert abhängig von einer der vorstehenden Zustandsgrößen des Gases nachgeführt werden. Falls das Modell keine Grenzsonde simuliert, sondern eine ausreichend schnelle Sonde, also beispielsweise eine Nominalsonde, gilt dies auch für den Überwachungsschwellwert.

Bei mangelnder Anregung kann prinzipiell der Fall eintreten, dass Zähler und/ oder Nenner des Energieverhältnisses einen Wert nahe oder gleich Null annehmen. Die Division durch Null kann abgefangen werden, indem man bei Änderung der zu messenden Gaszustandsgröße, beispielsweise bei Lastwechseln der Brennkraftmaschine, eine Prüfung auf ausreichende Anregung durchführt, wobei beim Modellsignal die Flankensteilheit, abhängig vom Vorzeichen der Signaländerung, mit einem applizierbaren Schwellwert verglichen wird. Alternativ kann die Prüfung auf ausreichende Anregung durchgeführt werden, indem die Flankensteilheit der Eingangsgrößen des Modells bewertet wird.

Als Erweiterung des Diagnoseverfahrens kann zusätzlich vorgesehen sein, dass eine iterative Identifikation einer Sensor-Zeitkonstante $T_S$ durchgeführt wird, wobei die Modell-Zeitkonstante $T_M$, abhängig vom Energieverhältnis der Signalanteile oder von einer davon abhängigen Größe in Schritten adaptiert wird. Dazu wird die Modell-Zeitkonstante $T_M$ mit einem Startwert $T_{init}$ initialisiert und in Schritten nach jeder Integration in Abhängigkeit des Energieverhältnisses korrigiert. Um die Konvergenz des Iterationsverfahrens zu beschleunigen oder zu verlangsamen, kann anstatt abhängig vom Energieverhältnis die Adaption abhängig von einer davon abhängigen Größe erfolgen.

In einer alternativen Verfahrensvariante kann eine Identifikation der Sensor-Zeitkonstante $T_S$ mittels in einem Kennfeld abgelegter Werte für die Zeitkonstante $T_S$ oder Verhältniswerte $T_S/T_M$ durchgeführt werden, wobei als Eingangsgrößen die Wertepaare Energieverhältnis und Modell-Zeitkonstante $T_M$ oder der Zähler und Nenner des jeweils berechneten Energieverhältnisses verwendet werden. Diese Methode ist insbesondere im Hinblick auf einen niedrigen Rechenaufwand von Vorteil und damit auch hinsichtlich der Zeit, bis ein Identifikationsergebnis vorliegt.

Das erfinderische Diagnoseverfahren kann besonders vorteilhaft bei Gas-Sensoren eingesetzt werden, die als Gas-Drucksensoren, Gas-Temperatursensoren, Gas-Massenstromsensoren oder Gaskonzentrationssensoren als Abgassonden im Abgaskanal der Brennkraftmaschine als Teil eines Abgasüberwachungs- und -minderungssystems oder in einer Zuluftführung der Brennkraftmaschine, z.B. im Ansaugkrümmer, verwendet werden, um Gaszustandsgrößen bzw. Konzentrationen zu erfassen. Diese emissionsrelevanten Gas-Sensoren müssen aufgrund der eingangs erwähnten Anforderungen hinsichtlich ihrer Dynamik und generellen Funktion überwacht werden. So kann z.B. das Ansprechverhalten eines Gas-Drucksensors überwacht werden und ein Nachlassen der Dynamik detektiert werden, wenn beispielsweise die Anbindung des Gas-Drucksensors an einen Ansaugkrümmer verstopft oder abgeknickt ist. Gas-Temperatursensoren oder Gas-Massenstromsensoren können beispielsweise als Heißfilm-Luftmassenmesser innerhalb einer Zuluftführung der Brennkraftmaschine ausgeführt sein und bei denen infolge einer Verschmutzung ein Dynamikverlust zu verzeichnen ist. Sofern für die Signale solcher Sensoren ein geeignetes Modell angegeben werden kann, kann das erfindungsgemäße Verfahren, wie zuvor in seinen Verfahrensvarianten beschrieben, vorteilhaft angewendet werden.

Als Gas-Sensoren kommen insbesondere Abgassonden in Form von Breitband-Lambdasonden (LSU-Sonden) oder $NO_x$-Sensoren in Betracht, mit denen ein Sauerstoffgehalt in einem Gasgemisch bestimmt werden kann. Für eine als Breitband-Lambdasonde oder stetige Lambdasonde ausgeführte Abgassonde wird zur Diagnose vorzugsweise die gemessene Sauerstoffkonzentration mit einer modellierten Sauerstoffkonzentration entsprechend den zuvor beschriebenen Verfahrensvarianten verglichen. Alternativ kann für diesen Vergleich der reziproke Lambdawert verwendet werden, da er näherungsweise proportional zur Sauerstoffkonzentration ist. Ebenfalls geeignet sind elektrische Größen, die proportional zur Sauerstoffkonzentration sind, d.h. eine Spannung oder ein Strom im Sensor bzw. im zugehörigen Schaltkreis. Das zum Vergleich herangezogene Modellsignal muss dann entsprechend umgerechnet werden. Für einen Stickoxid-Sensor wird als Istwert das Ausgangssignal des Stickoxid-Sensors ausgewertet, wobei der Modellwert aus einem modellierten $NO_x$-Wert bestimmt wird. Diese Diagnose lässt sich daher besonders vorteilhaft bei Otto-Motoren oder bei Mager-Motoren anwenden, deren Abgasreinigungsanlage einen Katalysator und/ oder Einrichtungen zur Stickoxid-Reduktion aufweisen. Bei Gas-Sensoren, die nach einer Abgasreinigungsanlage verbaut sind, muss der Einfluss der Abgasreinigung auf die interessierende Gaskonzentration im Modell berücksichtigt werden. Alternativ ist vorstellbar, die Diagnose nur in Phasen durchzuführen, in denen die Abgasreinigung keinen Einfluss auf die interessierende Gaskonzentration hat.

Eine weitere Anwendung des Verfahrens mit seinen zuvor beschriebenen Varianten kann generell bei Prozessen mit mindestens einem Sensor vorgesehen sein, bei denen sich der Prozess durch einen Filter erster Ordnung mit Zeitkonstante sowie ggf. mit einer Totzeit approximieren lässt und das Verhalten eines verlangsamten Sensors durch eine vergrößerte Filterzeitkonstante beschrieben werden kann. Grundsätzlich ergeben sich auch hierbei die zuvor beschrieben Vorteile hinsichtlich einer Beurteilung des Ansprechverhaltens des Sensors. Zudem kann ein solcher Prozess hinsichtlich seines Regelverhaltens verbessert werden, indem sein Regler an die veränderte Zeitkonstante angepasst wird.

Ein bevorzugte Anwendung des Verfahrens mit seinen zuvor beschrieben Varianten sieht den Einsatz bei Hybrid-Fahrzeugen, z.B. Diesel-Hybrid-Fahrzeugen, vor, welche keine Leerlauf-Betriebsphasen oder Schub-Phasen aufweisen. Besonders hervorzuheben ist, dass die Anwendung bei Hybrid-Fahrzeugen das Verbrauchs- und $CO_2$-Reduktionspotential nicht schmälert, da weder Testeinspritzungen nötig sind noch spezielle Motorbetriebszustände angefordert werden müssen.

Eine weitere bevorzugte Anwendung des Verfahrens mit seinen zuvor beschrieben Varianten sieht den Einsatz bei Fahrzeugen vor, die einen sogenannten Segelbetrieb aufweisen. Bei derartigen Fahrzeugen entfällt der Schubbetrieb ebenfalls weitestgehend. Statt den Motor im Schub zu schleppen, wird beim Segelbetrieb die Kupplung geöffnet, der Motor geht in den Leerlauf und das Fahrzeug rollt aufgrund seiner Trägheit. Auch hierbei ergeben sich erhebliche Verbrauchs- und $CO_2$-Reduktionspotentiale.

Zur Durchführung des erfindungsgemäßen Verfahrens kann eine Diagnoseeinheit welche jedoch nicht unter den Schutzumfang der beigefügte Ansprüche fällt, vorgesehen sein, die Hochpassfilter zur Auswertung höherfrequenter Signalanteile bei einer Änderung der zu messenden Gaszustandsgröße sowie mindestens ein Modell für den Sensor mit einem Tiefpassverhalten und Berechnungseinheiten, wie beispielsweise Integrationseinheiten, Komparatoren und ggf. Kennfeldeinheiten zur Durchführung der Dynamikdiagnose gemäß den zuvor beschriebenen Verfahrensvarianten aufweist. Die Funktionalität der Diagnoseeinheit kann dabei zumindest teilweise Software-basiert ausgeführt sein, wobei diese als separate Einheit oder als Teil einer übergeordneten Motorsteuerung vorgesehen sein kann.

Die Erfindung wird im Folgenden anhand eines in den Figuren dargestellten Ausführungsbeispiels näher erläutert. Es zeigen:

Figur 1 in schematischer Darstellung das technische Umfeld, in dem das erfindungsgemäße Verfahren angewendet werden kann,
Figur 2a und 2b jeweils Bode-Diagramme für einen schnellen und einen langsamen Gas-Sensor,
Figur 3 ein Blockdiagramm einer Dynamikdiagnoseschaltung gemäß der Erfindung,
Figur 4 ein Verlaufsdiagramm für unterschiedliche Signalpfade und
Figur 5 ein Flussdiagramm für eine iterative Identifikation einer Sonden-Zeitkonstante Ts.

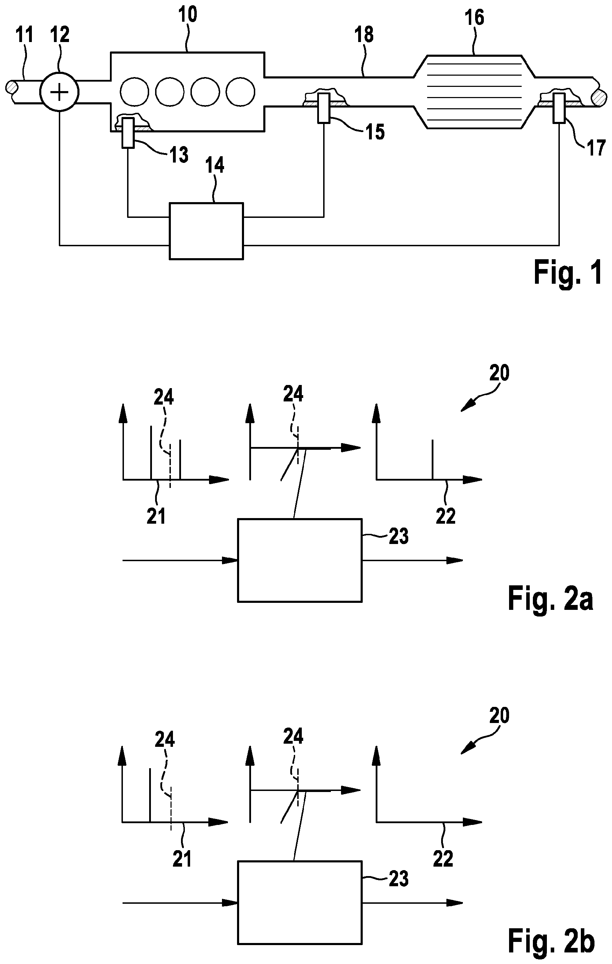

Figur 1 zeigt schematisch an einem Beispiel eines Otto-Motors das technische Umfeld, in dem das erfindungsgemäße Verfahren zur Diagnose einer Abgassonde 15 eingesetzt werden kann. Einer Brennkraftmaschine 10 wird Luft über eine Luftzuführung 11 zugeführt und deren Masse mit einem Luftmassenmesser 12 bestimmt. Der Luftmassenmesser 12 kann als Heißfilm-Luftmassenmesser ausgeführt sein. Das Abgas der Brennkraftmaschine 10 wird über einen Abgaskanal 18 abgeführt, wobei in Strömungsrichtung des Abgases hinter der Brennkraftmaschine 10 eine Abgasreinigungsanlage 16 vorgesehen ist. Die Abgasreinigungsanlage 16 umfasst üblicherweise mindestens einen Katalysator.

Zur Steuerung der Brennkraftmaschine 10 ist eine Motorsteuerung 14 vorgesehen, die zum einen der Brennkraftmaschine 10 über eine Kraftstoffdosierung 13 Kraftstoff zuführt und der zum anderen die Signale des Luftmassenmessers 12 und der in dem Abgaskanal 18 angeordneten Abgassonde 15 sowie einer in der Abgasableitung 18 angeordneten Abgassonde 17 zugeführt werden. Die Abgassonde 15 bestimmt im gezeigten Beispiel einen Lambda-Istwert eines der Brennkraftmaschine 10 zugeführten Kraftstoff-Luft-Gemischs. Sie kann als Breitband-Lambdasonde oder stetige LambdaSonde ausgeführt sein. Die Abgassonde 17 bestimmt die Abgaszusammensetzung nach der Abgasreinigungsanlage 16. Die Abgassonde 17 kann als Sprungsonde oder Binärsonde ausgebildet sein.

Hinsichtlich einer verbesserten Dynamiküberwachung der Abgassonde 15 ist gemäß der Erfindung vorgesehen, dass Hoch- und Tiefpassfilter genutzt werden, um bei einem Lastwechsel der Brennkraftmaschine 10 zu prüfen, ob die höherfrequenten Anteile einer Konzentrationsänderung von der Abgassonde 15 noch erkannt werden. Derartige Gas-Sensoren weisen ein typisches Tiefpassverhalten auf, das u.a. von der Geometrie ihres Schutzrohres abhängt. Bei Dieselmotoren kann zudem ein solches Schutzrohr verrußen, wodurch die Bandbreite des Sensors abnimmt. Im Zeitbereich äußert sich die abnehmende Grenzfrequenz in einer größeren Anstiegszeit, d.h. bei unveränderter Anregung werden die Signalflanken flacher. Schaltet man daher mit der Sonde einen geeigneten Hochpassfilter in Reihe, dann kann man bei steilen Lastwechseln am Ausgangssignal des Hochpasses erkennen, ob die Grenzfrequenz des Tiefpasses größer oder kleiner als die Grenzfrequenz des Hochpasses ist.

Die Figuren 2a und 2b zeigen schematisch anhand von Bode-Diagrammen 20 das Funktionsprinzip. Dargestellt sind im Frequenzbereich ein vereinfacht als Linienspektrum ausgeprägtes Eingangsspektrum 21 mit zwei Frequenzanteilen sowie einen Hochpass 1. Ordnung 23, dessen Übertragungsfunktion durch die Beziehung

$$G(j\omega) = T_F / (T_F \, j\omega +1) \qquad (1)$$

beschrieben werden kann mit $T_F$ als Grenzfrequenz des Filters (Filter-Grenzfrequenz 24). Übersteigt die Grenzfrequenz der Abgassonde 15 die Grenzfrequenz $T_F$ des Hochpasses 1. Ordnung 23, dann verhält sich die Serienschaltung wie ein Bandpass, d.h. die hohen Frequenzen des Eingangsspektrums 21 der Abgassonde 15 werden noch durchgelassen und können im Ausgangsspektrum 22 detektiert werden, wie dies

Figur 2a schematisch zeigt. Fällt dagegen die Grenzfrequenz der Abgassonde 15 infolge eines Dynamikverlustes unter die Grenzfrequenz $T_F$ des Hochpasses 1. Ordnung 23 (Filter-Grenzfrequenz 24), dann sperrt die Reihenschaltung sämtliche Frequenzen, so dass im Ausgangsspektrum 22 keinerlei Frequenzanteile mehr gemessen werden können (Figur 2b). Es sei angemerkt, dass dieses Linienspektrum lediglich zur Erläuterung des Prinzips dient. Das reale Frequenzspektrum einer Abgassonde 15 lässt sich durch kontinuierlich verlaufende Frequenzanteile beschreiben.

Prinzipiell beschränkt sich die Erfindung dabei nicht auf Hochpassfilter 1. Ordnung.

Vielmehr können auch beliebig andere Hochpassfilter zum Einsatz kommen. Ebenso ist die Überwachungsmethode anwendbar, wenn die Tiefpassfilter einschließlich der Abgassonde 15 selbst anders parametriert werden, z.B. mit einer Grenzfrequenz anstelle der Zeitkonstanten, oder eine höhere Ordnung besitzen.

Damit zwischen einer langsamen Abgassonde 15 und einer unzureichenden Anregung unterschieden werden kann, muss die Änderungsgeschwindigkeit der Abgaszusammensetzung beurteilt werden, was beispielsweise im Falle einer Breitband-Lambdasonde anhand von Luft- und Kraftstoffmassenänderung geschehen kann. Dies kann mit einer ähnlichen Reihenschaltung von Filtern geschehen. Im Falle einer Breitband-Lambdasonde müssen hierfür nur die vorstehenden Massen in eine $O_2$-Konzentration umgerechnet und mit einem Tiefpassfilter verzögert werden, der einem funktionsfähigen Abgassensor entspricht. Dieser Tiefpassfilter ist dann mit einem Hochpass in Reihe zu schalten, der die gleiche Übertragungsfunktion besitzt wie der der realen Sonde. Durch einen Vergleich der beiden Hochpass-Ausgänge kann dann auf die Funktionstüchtigkeit des realen Sensors geschlossen werden. Im Falle einer anderen Gaskomponente kann es erforderlich werden, ein zusätzliches Rohemissionsmodell zu verwenden.

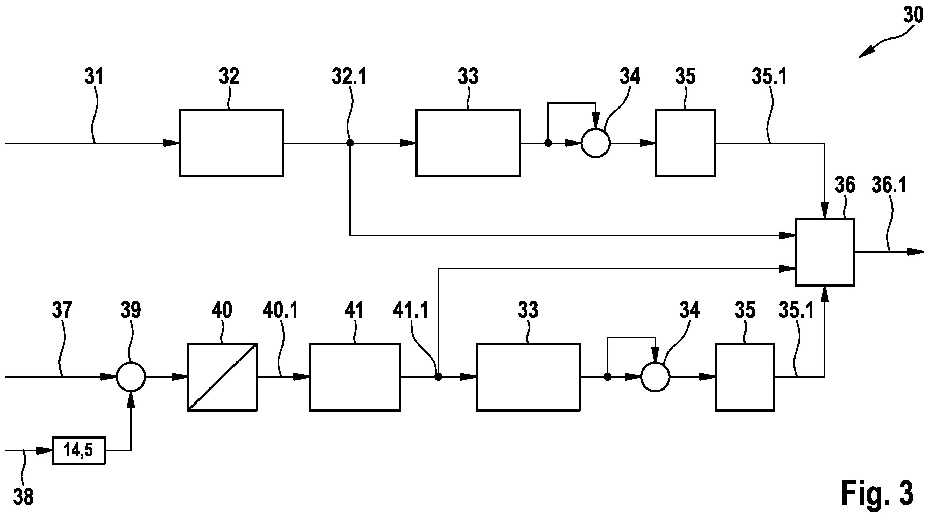

Figur 3 zeigt in einem Blockdiagramm 30 die Funktionalität des zuvor beschriebenen Prinzips in einer bevorzugten Verfahrensvariante. Dargestellt ist im oberen Teil ein Pfad für eine mit der Abgassonde 15 gemessenen Sauerstoffkonzentration 31. Infolge einer realen Gaslaufzeit und Sondenverzögerung 32, welche durch eine Totzeit $T_t$ bzw. einen Tiefpassfilter 1. Ordnung mit einer Sonden-Zeitkonstante $T_S$ beschrieben werden können, ergibt sich aus der realen Sauerstoffkonzentration 31 ein Sauerstoff-Sondensignal 32.1. Die Übertragungsfunktion der Sonden und- Gaslaufzeit 32 ergibt sich durch folgende Beziehung, wobei $K_S$ einen Verstärkungsfaktor für die Sonde darstellt:

$$G(j\omega) = K_S \, \exp(-T_t \, j\omega) / (T_s \, j\omega +1) \qquad (2)$$

$K_S$ entspricht in der Regel dem multiplikativen oder Steigungsfehler der Sonde, der von Produktionsstreuung und Alterung herrührt. Wird jedoch als Sondensignal nicht die Sauerstoffkonzentration verwendet, sondern eine dazu proportionale Größe, dann ist $K_S$ ein entsprechender Übertragungsbeiwert zur Umrechnung des Sondensignals in eine Sauerstoffkonzentration und kann auch dimensionsbehaftet sein. Anschließend wird das Sauerstoff-Sondensignal 32.1 mit einem Hochpass 33, dessen Übertragungsfunktion der des Hochpasses 1. Ordnung 23 aus Figur 2a bzw. 2b entspricht, gefiltert und mit einem Multiplizierer 34 quadriert, was ein Signal liefert, welches einer Signalleistung entspricht. Dieses Signal wird anschließend mittels eines Integrators 35 integriert, so dass man eine Signalenergie 35.1 der höherfrequenten Energieanteile des gemessenen Sauerstoffgehaltes erhält. In einer nachgeschalteten Normierungs- und Divisionseinheit 36 ergibt sich aus einem Vergleich mit einem entsprechend aufbereiteten Signal für einen modellhaft bestimmten Wert ein Energieverhältnis 36.1 E, welches ein Anteilsmaß für die höherfrequenten Energieanteile ist.

Die Aufbereitung des modellhaft bestimmten Energiewertes wird im unteren Teil des Blockdiagramms 30 gezeigt. Aus einer Luftmasse 37 $m_L$ und einer Soll-Kraftstoffmasse 38 $m_K$ für die Kraftstoffdosierung 13 wird nach stöchiometrischer Korrektur in einer Divisionseinheit 39 ein Quotient gebildet und ein Lambdawert errechnet. Die Kraftstoffmasse 38 kann sich aus dem Drehmomentwunsch ergeben, den der Fahrer vorgibt und der in eine Kraftstoffmenge umgerechnet wird. In einer Umrechnungseinheit 40 wird aus dem Lambdawert ein kalkulierter Sauerstoffgehalt 40.1 bestimmt. Gemäß einem Modell 41 wird mit der Übertragungsfunktion

$$G(j\omega) = \exp(-T_{tM}\, j\omega) \,/\, (T_M\, j\omega + 1) \qquad (3)$$

ein modellierter Sauerstoffgehalt 41.1 berechnet, wobei $T_{tM}$ eine Modell-Totzeit und $T_M$ eine Modell-Zeitkonstante darstellt.

Anschließend wird der modellierte Sauerstoffgehalt 41.1 mit einem weiteren Hochpass 33, dessen Übertragungsfunktion der des Hochpasses 1. Ordnung 23 aus Figur 2a bzw. 2b entspricht, gefiltert und mit einem weiteren Multiplizierer 34 quadriert, was ein Signal liefert, welches einer Signalleistung entspricht. Dieses Signal wird anschließend mittels eines weiteren Integrators 35 integriert, so dass man eine Signalenergie 35.1 für die höherfrequenten Energieanteile des modellieren Sauerstoffgehalts erhält.

Die Integration des quadrierten Signals liefert ein Maß für die Energie der höherfrequenten Anteile des jeweiligen $O_2$-Signals 32.1, 41.1. Je kleiner die Fläche unter dem quadrierten Ausgangssignal des Hochpasses 33 ist, desto langsamer ist die Sonde bzw. die Anregung. Alternativ zu den Signalenergien können auch Größen gebildet und ins Verhältnis gesetzt werden, die eng mit den Signalenergien zusammenhängen. Beispielsweise kann statt der Signalenergie auch die Wurzel der Signalenergie verwendet werden oder der Betrag des Hochpass-Ausgangssignals integriert werden. Das Verhältnis derartiger Ersatzgrößen muss dann 1 sein, wenn das Energieverhältnis 1 ist, d.h. wenn das Dynamikverhalten von Gas-Sensor und Modell gleich ist.

Damit multiplikative Fehler des Gas-Sensors und/ oder des Modells bzw. seiner Eingangssignale den Signalvergleich nicht verfälschen, werden sie durch Normierung wie folgt weitgehend eliminiert. Das Energieverhältnis 36.1 E lässt sich demnach wie folgt darstellen:

$$E = \frac{(Um(t1+\Delta t)-Um(t1))^2 \int_{t0}^{t0+\Delta t}(Ys(t))^2\, dt}{(Us(t0+\Delta t)-Us(t0))^2 \int_{t1}^{t1+\Delta t}(Ym(t))^2\, dt} \qquad (4)$$

Darin repräsentieren $U_s$ und $Y_s$ den Hochpassein- bzw. -ausgang im Signalpfad der Sonde und $U_m$ und $Y_m$ deren Pendants im Modellpfad. D.h. $U_s$ entspricht dem Sauerstoff-Sondensignal 32.1 und $U_m$ dem kalkulierten Sauerstoffgehalt 40.1 aus dem Modell. Durch die Verhältnisbildung wird der Einfluss des Signalhubs der Anregung ebenfalls eliminiert.

Die Integration in den beiden Signalpfaden muss nicht unbedingt simultan gestartet werden, sondern kann derart gestartet werden, dass unterschiedliche Totzeiten das Diagnoseergebnis nicht beeinflussen.

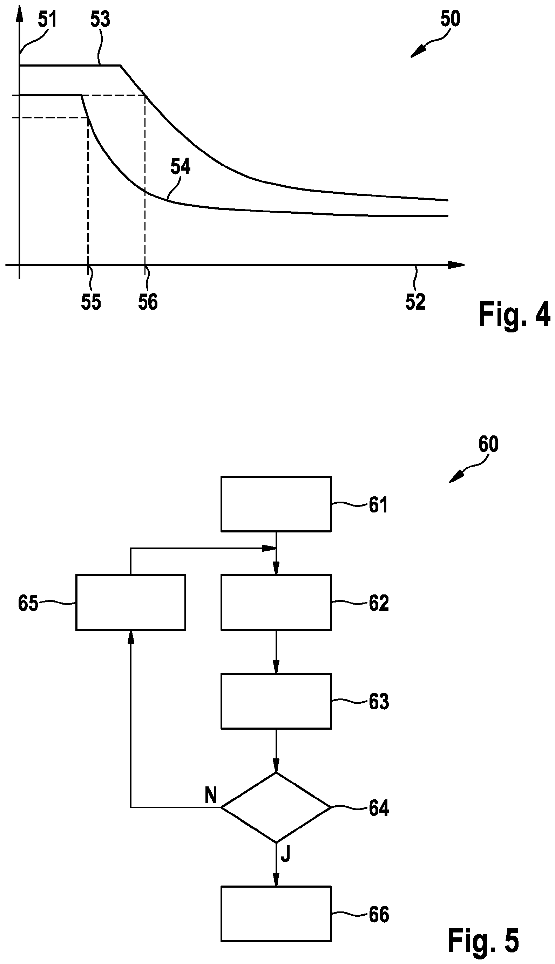

Figur 4 zeigt in einem Verlaufsdiagramm 50 exemplarisch die Zeitpunkte 55, 56 $t_1$, $t_0$ für den Start der Integration bei fallender Flanke. Dargestellt ist die Signalhöhe in Abhängigkeit von der Zeit 52 für einen Signalverlauf Sensor 53 und für einen Signalverlauf Modell 54. Ausgehend von einem bestimmten Stationärpunkt wird bei Einsetzen einer Signalflanke erst ein gewisser Signalhub zurückgelegt, bevor die Integration gestartet wird. Die individuelle Integrationsdauer kann für beide Pfade unterschiedlich lang sein. Weiterhin kann vorgesehen sein, dass bei steigender Signalflanke die Integration gestartet wird, wobei dann entsprechend vorgegangen wird. Ein kurzzeitiger Stationärbetrieb vor der Überwachung stellt sicher, dass die Signalflanken von Sonde und Modell auch bei Totzeitunterschieden von derselben Anregung herrühren. Dies ist insbesondere dann wichtig, wenn der Einbauort der Abgassonde 15 manipuliert wurde.

Falls steigende und fallende Flanken des Sensorsignals nicht getrennt überwacht werden müssen, d.h. wenn kein sogenanntes Pinpointing erforderlich ist, kann das erfindungsgemäße Verfahren an sich vereinfacht werden. Es ist dann alternativ möglich, die Integration in beiden Pfaden zu einem beliebigen Zeitpunkt $t_0$ zu starten und für die Dauer $\Delta t$ auszuführen. Voraussetzung ist lediglich eine ausreichende Anregung durch Änderung der zu messenden Gaskonzentration bzw. Zustandsgröße, beispielsweise infolge wechselnder Motorbetriebspunkte. Das Zeitintervall $\Delta t$ darf also mehrere fallende und steigende Flanken umfassen. Für die Normierung ist dann das jeweilige Maximum und Minimum während $\Delta t$ an beiden Hochpasseingängen zu erfassen. Das Energieverhältnis 36.1 E für die Diagnose und/ oder Identifikation lautet dann:

$$E = \frac{(Um,\max - Um,\min)^2 \int_{t0}^{t0+\Delta t}(Ys(t))^2\, dt}{(Us,\max - Us,\min)^2 \int_{t0}^{t0+\Delta t}(Ym(t))^2\, dt} \qquad (5)$$

mit

$U_{s/m}$ = O$_2$-Konzentration gemessen mit der Sonde/ aus dem Modell bestimmt
$Y_{s/m}$ = Hochpass-Ausgangssignal Sonde/Modell
$U_{s/m,max}$ = Maximum des Hochpass-Eingangsignals im Int
$U_{s/m,min}$ = Minimum des Hochpass-$_{tM}$, $T_t$, d.h. Integrationsdauer >> Modelltotzeit $T_{tM}$ bzw. Sonden-Totzeit $T_t$

Aus dem Wert des Energieverhältnisses 36.1 E kann nun wie folgt auf einen Dynamikfehler der Abgassonde 15 geschlossen werden: Wenn die obige Division der Signalenergien 35.1 auf einen Wert größer als eins führt, dann ist die reale Sonde schneller als das Modell. Ist der Bruch kleiner als eins, dann ist die reale Sonde langsamer als das Modell.

Das obige Energieverhältnis 36.1 E wird nun mit einem Schwellwert verglichen, der die Dynamik einer Grenzsonde repräsentiert. Dieser Schwellwert hängt davon ab, wie die Modellzeitkonstante $T_M$ gewählt wurde. Entspricht $T_M$ bereits einer grenzwertigen Sensordynamik, dann liegt der passende Schwellwert bei 1, so dass seine Applikation entfällt. Entspricht die Modell-Zeitkonstante $T_M$ dagegen einer ausreichend schnellen Gas-Sonde, z.B. einer Nominal-Sonde, dann ist der passende Schwellwert kleiner als 1. Für den Fall, dass das Energieverhältnis E 36.1 E $\geq$ dem Schwellwert ist, ist die Abgassonde 15 als i.O. zu betrachten, im Fall, dass das Energieverhältnis 36.1 E $\leq$ dem Schwelllwert ist, ist die Abgassonde 15 als fehlerhaft zu betrachten.

Bei mangelnder Anregung kann prinzipiell der Fall eintreten, dass Zähler und/ oder Nenner von E in der Gleichung (4) zu Null werden. Die Division durch Null kann abgefangen werden, in dem man eine Prüfung auf ausreichende Anregung durchführt. Hierfür kann man z.B. den Modellpfad, ggf. zusätzlich, für eine Nominalsonde implementieren und prüfen, ob der kalkulierte Sauerstoffgehalt 40.1 bzw. der modellierte Sauerstoffgehalt 41.1 eine ausreichende Flankensteilheit aufweist. In einer bevorzugten Variante dieser Prüfung wird festgestellt, ob der zugehörige Hochpassausgang bei positiven Flanken für den kalkulierten Sauerstoffgehalt 40.1 bzw. für den modellierten Sauerstoffgehalt 41.1 einen gewissen positiven Schwellwert überschreitet oder bei negativen Flanken einen bestimmten negativen Schwellwert unterschreitet.

Die Erfindung kann erweitert werden, so dass damit auch eine iterative Identifikation der Sonden-Zeitkonstante $T_S$ möglich ist. Figur 5 zeigt in einem Flussdiagramm 60 den funktionellen Ablauf.

Hierzu wird in einer Initialisierungseinheit 61 die Modell-Zeitkonstante $T_M$ mit einem Startwert $T_{init}$ initialisiert und dann nach jeder Integration in Abhängigkeit des Energieverhältnisses 36.1 E korrigiert. Dazu wird in der Funktionseinheit 62 ein Wert $E_k$ durch Messen und Auswerten bestimmt. Es gilt:

$$k = 1, \qquad T_{M,k} = T_{init} \qquad\qquad (6)$$

Ist $T_M$ größer als der gesuchte Wert $T_S$, dann ist die Sonde schneller als das Modell und das Energieverhältnis 36.1 E größer als eins. In diesem Fall wird $T_M$ im nächsten Schritt verkleinert, indem man durch E dividiert, was in der Berechnungseinheit 63 durchgeführt wird. Im Falle $T_M < T_S$ ist $E < 1$ und die nachfolgende Division $T_M / E$ hebt die neue Modell-Zeitkonstante $T_{M,k+1}$ an. Die Iterationsvorschrift lautet damit:

$$T_{M,1} \qquad = T_{init} \qquad\qquad (7a)$$

$$T_{M,k+1} \qquad = T_{M,k} / E_k \qquad\qquad (7b)$$

wobei k den k-ten Iterationsschritt bezeichnet. Im Laufe der Integration konvergiert $E_k$ gegen 1 und $T_{M,k}$ gegen $T_S$. Dabei ist es unerheblich, ob $T_{M,k}$ streng monoton gegen $T_S$ strebt oder mit abnehmender Amplitude um $T_S$ herum oszilliert. Das Entsprechende gilt für $E_k$. Die Iteration wird beendet, wenn

$$1 - \varepsilon < E_k < 1 + \varepsilon \quad \text{bzw.} \quad |E_k - 1| < \varepsilon \qquad (8)$$

ist. Diese Überprüfung geschieht mittels der Abfrage 64. Ist die zuvor genannte Bedingung erfüllt, wird als Ergebnis 66 der Iteration $T_S = T_{M,k+1}$ ausgegeben. Ist die Bedingung noch nicht erfüllt, wird ein Zähler 65 für k um Eins erhöht.

Die iterative Identifikation kann on-line durchgeführt werden, wobei dann in jedem Iterationsschritt eine andere Anregung erfolgt. Ebenso kann die Iteration off-line durchgeführt werden, wobei dann dieselbe Messung mehrmals ausgewertet wird. Wird die Erfindung auf diese Weise für die Identifikation genutzt, dann muss zur Sensordiagnose der Endwert von $T_{M,k}$ mit einer grenzwertigen Zeitkonstante verglichen werden. Ein Vergleich von E mit 1 ist dann offenkundig nicht mehr sinnvoll.

Die Konvergenz des Identifikationsverfahrens kann beschleunigt oder auch verlangsamt werden, indem für die Berechnung von $T_{M,k+1}$ nicht $E_k$ selbst, sondern eine davon abhängige Größe verwendet wird. Das iterative Identifikationsverfahren sucht die Lösung $T_M = T_S$ der Gleichung $E(T_M) = 1$. Für die iterative Identifikation können alternativ andere numerische Verfahren zur Lösung von sogenannten Fixpunktgleichungen herangezogen werden.

Alternativ kann als Einfachansatz für eine Sensoridentifikation auch ein Kennfeld für die geschätzte Zeitkonstante $T_S$ aufgespannt werden. Als Eingänge für dieses Kennfeld kommen folgende Kombinationen von Größen in Betracht: $T_M$ und E oder Zähler und Nenner von E (vergleiche (4) und (5)). Im Kennfeld kann $T_S$ oder das Verhältnis $T_S/T_M$ abgelegt sein.

**Patentansprüche**

1.  Verfahren zur Dynamiküberwachung von Gas-Sensoren einer Brennkraftmaschine (10), wobei die Gas-Sensoren abhängig von Geometrie, Messprinzip, Alterung oder Verschmutzung ein Tiefpassverhalten aufweisen, das durch eine Grenzfrequenz der Abgassonde (15) beschrieben wird, wobei bei einer Änderung der zu messenden Gaszustandsgröße auf Grund eines Vergleiches eines modellierten und eines gemessenen Signals eine Dynamikdiagnose durchgeführt wird und wobei das gemessene Signal ein Istwert eines Ausgangssignals des Gas-Sensors und das modellierte Signal ein Modellwert ist, wobei das Ausgangssignal des Gas-Sensors mit einem geeigneten Hochpassfilter (23, 33) gefiltert wird, der durch die Grenzfrequenz ($T_F$) eines Hochpasses erster Ordnung (23) beschrieben wird, und bei einer Änderung der zu messenden Gaszustandsgröße höherfrequente Signalanteile ausgewertet werden, und ein Dynamikverlust vorliegt, wenn die Grenzfrequenz der Abgassonde (15) unter die Grenzfrequenz ($T_F$) des Hochpasses erster Ordnung (23) fällt, wobei bei einer Änderung eines Luft-Kraftstoff-Verhältnisses eines der Brennkraftmaschine (10) zugeführten Luft-Kraftstoffgemischs die Dynamikdiagnose des Gas-Sensors durchgeführt wird und bei dieser Änderung des Luft-Kraftstoff-Verhältnisses der Brennkraftmaschine (10) höherfrequente Signalanteile ausgewertet werden, wobei die höherfrequenten Signalanteile des Gas-Sensors mit entsprechend hochpassgefilterten Ausgangssignalen aus einem Modell (41) des Gas-Sensors verglichen werden und anhand des Vergleichs auf die Dynamik des Gas-Sensors geschlossen wird, wobei sowohl die höherfrequenten Signalanteile des Gas-Sensors als auch die des Modells (40) quadriert sowie integriert und damit höherfrequente Energieanteile berechnet werden, und anschließend diese Energieanteile ins Verhältnis gesetzt werden und anhand des derart berechneten Energieverhältnisses (36.1) auf das Dynamikverhalten des Gas-Sensors geschlossen wird.

2.  Verfahren nach Anspruch 1, **dadurch gekennzeichnet, dass** eine Normierung der jeweiligen Energieanteile durchgeführt wird.

3.  Verfahren nach Anspruch 1 oder 2, **dadurch gekennzeichnet, dass** die Integration der höherfrequenten Signalanteile mittels einer für beide Signale individuellen Integrationsdauer $\Delta t$ durchgeführt wird, wobei der Zeitpunkt (55, 56) für den Start der Integration der beiden Signale sowohl bei einer ansteigenden Signalflanke des Ausgangssignals als auch bei einer fallenden Signalflanke getriggert wird.

4.  Verfahren nach Anspruch 3, **dadurch gekennzeichnet, dass**, ausgehend von einem bestimmten Stationärpunkt, bei Einsetzen der Signalflanken ein applizierbarer Signalhub abgewartet wird, bevor die Integration gestartet wird.

5.  Verfahren nach einem der Ansprüche 1 bis 4, **dadurch gekennzeichnet, dass** das Energieverhältnis (36.1) mit einem applizierbaren Schwellwert verglichen wird, der die Dynamik eines grenzwertigen Sensors repräsentiert, wobei der Schwellwert und eine im Modell (41) für den Sensor gewählte Modell-Zeitkonstante $T_M$ voneinander abhängig sind.

6.  Verfahren nach einem der Ansprüche 1 bis 5, **dadurch gekennzeichnet, dass** Filterzeitkonstanten und/ oder der Schwellwert abhängig von Zustandsgrößen des Gases nachgeführt werden.

7.  Verfahren nach einem der Ansprüche 1 bis 6, **dadurch gekennzeichnet, dass** bei Änderungen der zu messenden Gaszustandsgröße eine Prüfung auf eine ausreichende Anregung durchgeführt wird, wobei beim Modellsignal die Flankensteilheit, abhängig vom Vorzeichen der Signaländerung, mit einem applizierbaren Schwellwert verglichen wird.

8.  Verfahren nach einem der Ansprüche 1 bis 7, **dadurch gekennzeichnet, dass** eine iterative Identifikation einer Sensor-Zeitkonstante Ts durchgeführt wird, wobei die Modell-Zeitkonstante $T_M$, abhängig vom Energieverhältnis (36.1) der Signalanteile oder von einer davon abhängigen Größe in Schritten adaptiert wird.

9. Verfahren nach einem der Ansprüche 1 bis 8, **dadurch gekennzeichnet, dass** eine Identifikation der Sensor-Zeitkonstante Ts mittels in einem Kennfeld abgelegter Werte für die Zeitkonstante $T_S$ oder Verhältniswerte $T_S/T_M$ durchgeführt wird, wobei als Eingangsgrößen die Wertepaare Energieverhältnis (36.1) und Modell-Zeitkonstante $T_M$ oder der Zähler und Nenner des jeweils berechneten Energieverhältnisses (36.1) verwendet werden.

10. Verfahren nach einem der Ansprüche 1 bis 9, **dadurch gekennzeichnet, dass** als Gas-Sensoren Gas-Drucksensoren, Gas-Temperatursensoren, Gas-Massenstromsensoren oder Gaskonzentrationssensoren als Abgassonden (15) im Abgaskanal (18) der Brennkraftmaschine (10) als Teil eines Abgasüberwachungs- und -minderungssystems oder in einer Zuluftführung (11) der Brennkraftmaschine (10) verwendet werden.

11. Verfahren nach einem der Ansprüche 1 bis 10, **dadurch gekennzeichnet, dass** als Gas-Sensoren Abgassonden (15) in Form von Breitband-Lambdasonden oder $NO_x$-Sensoren verwendet werden, mit denen ein Sauerstoffgehalt in einem Gasgemisch bestimmt werden kann.

12. Anwendung des Verfahrens gemäß den Ansprüchen 1 bis 11 bei Hybrid-Fahrzeugen.

13. Anwendung des Verfahrens gemäß den Ansprüchen 1 bis 11 bei Fahrzeugen mit Segelbetrieb.

**Claims**

1. Method for monitoring the dynamics of gas sensors of an internal combustion engine (10), wherein, depending on the geometry, measurement principle, ageing or soiling, the gas sensors have a low-pass behaviour which is described by a cut-off frequency of the exhaust gas probe (15), wherein, if the gas state variable to be measured changes, the dynamics are diagnosed on the basis of a comparison of a modelled signal and a measured signal, and wherein the measured signal is an actual value of an output signal from the gas sensor and the modelled signal is a model value, wherein the output signal from the gas sensor is filtered using a suitable high-pass filter (23, 33) which is described by the cut-off frequency ($T_F$) of a first-order high-pass filter (23), and, if the gas state variable to be measured changes, higher-frequency signal components are evaluated, and there is a loss of dynamics if the cut-off frequency of the exhaust gas probe (15) falls below the cut-off frequency ($T_F$) of the first-order high-pass filter (23), wherein, if an air/fuel ratio of an air/fuel mixture supplied to the internal combustion engine (10) changes, the dynamics of the gas sensor are diagnosed, and, in the event of this change in the air/fuel ratio of the internal combustion engine (10), higher-frequency signal components are evaluated, wherein the higher-frequency signal components of the gas sensor are compared with correspondingly high-pass-filtered output signals from a model (41) of the gas sensor and the dynamics of the gas sensor are inferred on the basis of the comparison, wherein both the higher-frequency signal components of the gas sensor and those of the model (40) are squared and integrated and are used to calculate higher-frequency energy components, and these energy components are then related and the dynamic behaviour of the gas sensor is inferred on the basis of the energy ratio (36.1) calculated in this manner.

2. Method according to Claim 1, **characterized in that** the respective energy components are standardized.

3. Method according to Claim 1 or 2, **characterized in that** the higher-frequency signal components are integrated by means of an individual integration period $\Delta t$ for both signals, wherein the time (55, 56) for starting the integration of the two signals is triggered both in the case of a rising signal edge of the output signal and in the case of a falling signal edge.

4. Method according to Claim 3, **characterized in that**, starting from a particular steady-state point, an applicable signal swing is waited for when using the signal edges before the integration is started.

5. Method according to one of Claims 1 to 4, **characterized in that** the energy ratio (36.1) is compared with an applicable threshold value representing the dynamics of a borderline sensor, wherein the threshold value and a model time constant $T_M$ selected in the model (41) for the sensor are dependent on one another.

6. Method according to one of Claims 1 to 5, **characterized in that** filter time constants and/or the threshold value are tracked on the basis of state variables of the gas.

7. Method according to one of Claims 1 to 6, **characterized in that**, in the event of changes in the gas state variable to be measured, a check for sufficient excitation is carried out, wherein, depending on the sign of the signal change, the edge steepness of the model signal is compared with an applicable threshold value.

8. Method according to one of Claims 1 to 7, **characterized in that** a sensor time constant $T_S$ is iteratively identified, wherein the model time constant $T_M$ is adapted in increments on the basis of the energy ratio (36.1) of the signal components or a variable dependent thereon.

9. Method according to one of Claims 1 to 8, **characterized in that** the sensor time constant $T_S$ is identified by means of values for the time constant $T_S$ or ratio values $T_S/T_M$ that are stored in a characteristic map, wherein the value pairs of the energy ratio (36.1) and the model time constant $T_M$ or the numerator and denominator of the respectively calculated energy ratio (36.1) are used as input variables.

10. Method according to one of Claims 1 to 9, **characterized in that**, as gas sensors, gas pressure sensors, gas temperature sensors, gas mass flow sensors or gas concentration sensors are used as exhaust gas probes (15) in the exhaust gas duct (18) of the internal combustion engine (10) as part of an exhaust gas monitoring and reduction system or in an air intake line (11) of the internal combustion engine (10).

11. Method according to one of Claims 1 to 10, **characterized in that** exhaust gas probes (15) in the form of broadband lambda probes or $NO_x$ sensors are used as gas sensors and can be used to determine an oxygen content in a gas mixture.

12. Use of the method according to Claims 1 to 11 in hybrid vehicles.

13. Use of the method according to Claims 1 to 11 in vehicles with a coasting mode.


**Revendications**

1. Procédé permettant de surveiller la dynamique de capteurs de gaz d'un moteur à combustion interne (10), dans lequel les capteurs de gaz présentent indépendamment de la géométrie, du principe de mesure, du vieillissement ou de l'encrassement un comportement passe-bas qui est décrit par une fréquence limite de la sonde de gaz d'échappement (15), dans lequel un diagnostic de dynamique est effectué en cas de variation de la grandeur d'état de gaz à mesurer sur la base d'une comparaison d'un signal modélisé et d'un signal mesuré, et dans lequel le signal mesuré est une valeur réelle d'un signal de sortie du capteur de gaz, et le signal modélisé est une valeur modèle, dans lequel le signal de sortie du capteur de gaz est filtré par un filtre passe-haut (22, 23) approprié qui est décrit par la fréquence limite ($T_F$) d'un passe-haut du premier ordre (23), et en cas de variation de la grandeur d'état de gaz à mesurer, des composantes de signal de fréquence supérieure sont évaluées, et une perte de dynamique est avérée si la fréquence limite de la sonde de gaz d'échappement (15) descend sous la fréquence limite ($T_F$) du passe-haut du premier ordre (23), dans lequel, en cas de variation d'un rapport air/carburant d'un mélange air/carburant amené au moteur à combustion interne (10), le diagnostic de dynamique du capteur de gaz est effectué, et des composantes de signal de fréquence supérieure sont évaluées lors de cette variation du rapport air/carburant du moteur à combustion interne (10), dans lequel les composantes de signal de fréquence supérieure du capteur de gaz sont comparées avec des signaux de sortie filtrés passe-bas de façon correspondante, issus d'un modèle (41) du capteur de gaz, et la dynamique du capteur de gaz est déduite à l'aide de la comparaison, dans lequel, à la fois les composantes de signal du capteur de gaz et celles du modèle (40) sont élevées au carré et intégrées, et des composantes d'énergie de fréquence supérieure sont ainsi calculées, et ensuite ces composantes d'énergie sont mises en rapport, et le comportement dynamique du capteur de gaz est déduit à l'aide du rapport d'énergie (36.1) ainsi calculé.

2. Procédé selon la revendication 1, **caractérisé en ce qu'**une normalisation des composantes d'énergie respectives est effectuée.

3. Procédé selon la revendication 1 ou 2, **caractérisé en ce que** l'intégration des composantes de signal de fréquence supérieure est effectuée au moyen d'une durée d'intégration $\Delta t$ spécifique pour les deux signaux, dans lequel l'instant (55, 56) du départ de l'intégration des deux signaux est déclenché à la fois au niveau d'un flanc de signal croissant du signal de sortie et d'un flanc de signal décroissant.

4. Procédé selon la revendication 3, **caractérisé en ce qu'**en partant d'un point stationnaire déterminé, on attend à l'apparition des fronts de signal une déviation de signal applicable avant de commencer l'intégration.

5. Procédé selon l'une quelconque des revendications 1 à 4, **caractérisé en ce que** le rapport d'énergie (36.1) est comparé avec une valeur seuil applicable qui représente la dynamique d'un capteur douteux, dans lequel la valeur seuil et une constante de temps modèle $T_M$ sélectionnée pour le capteur dans le modèle dépendent l'une de l'autre.

6. Procédé selon l'une quelconque des revendications 1 à 5, **caractérisé en ce que** des constantes de temps de filtrage et/ou la valeur limite sont suivies en fonction des grandeurs d'état du gaz.

7. Procédé selon l'une quelconque des revendications 1 à 6, **caractérisé en ce qu'**en cas de variations de la grandeur d'état de gaz à mesurer, un contrôle d'une excitation suffisante est effectué, dans lequel pour le signal modèle, la pente de signal, en fonction du signe de la variation de signal, est comparée avec une valeur seuil applicable.

8. Procédé selon l'une quelconque des revendications 1 à 7, **caractérisé en ce qu'**une identification itérative d'une constante de temps de capteur $T_S$ est effectuée, dans lequel la constante de temps modèle $T_M$ est adaptée en fonction du rapport d'énergie (36.1) des composantes de signal ou d'une grandeur qui en dépend.

9. Procédé selon l'une quelconque des revendications 1 à 8, **caractérisé en ce qu'**une identification de la constante de temps de capteur $T_S$ est effectuée au moyen de valeurs stockées dans un diagramme caractéristique pour la constante de temps $T_S$ ou les valeurs de rapport $T_S/T_M$, dans lequel les paires de valeurs rapport d'énergie (36.1) et constante de temps modèle $T_M$ ou le numérateur et le dénominateur du rapport d'énergie (36.1) respectivement calculé sont utilises comme grandeurs d'entrée.

10. Procédé selon l'une quelconque des revendications 1 à 9, **caractérisé en ce que** des capteurs de pression de gaz, des capteurs de température de gaz, des capteurs de débit massique de gaz sont utilisés comme capteurs de gaz, ou des capteurs de concentration de gaz sont utilisés comme sondes de gaz d'échappement (15) dans le canal de gaz d'échappement (18) du moteur à combustion interne (10) faisant partie d'un système de surveillance et de réduction des gaz d'échappement ou dans une arrivée d'air frais (11) du moteur à combustion interne (10).

11. Procédé selon l'une quelconque des revendications 1 à 10, **caractérisé en ce que** des sondes de gaz d'échappement (15) sous forme de sondes lambda à large bande ou des capteurs $NO_X$ sont utilisés comme capteurs de gaz et permettent de déterminer une teneur en oxygène dans un mélange gazeux.

12. Application du procédé selon l'une quelconque des revendications 1 à 11 sur des véhicules hybrides.

13. Procédé selon l'une des revendications 1 à 11 sur des véhicules fonctionnant sans friction.

Fig. 1

Fig. 2a

Fig. 2b

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008001121 A1
- DE 102008040737 A1
- DE 102008001569 A1
- DE 102008042549 A1